(19) Europäisches Patentamt
European Patent Office
Office européen des brevets

(11) Publication number: **0 238 331 B1**

(12) # EUROPEAN PATENT SPECIFICATION

(45) Date of publication of patent specification: **13.12.89**

(51) Int. Cl.[4]: **C 07 C 11/09**, C 07 C 29/16

(21) Application number: **87302352.7**

(22) Date of filing: **18.03.87**

(54) **Dual step process for producing isobutylene from propylene and synthesis gas.**

(30) Priority: **18.03.86 FI 861133**

(43) Date of publication of application:
**23.09.87 Bulletin 87/39**

(45) Publication of the grant of the patent:
**13.12.89 Bulletin 89/50**

(84) Designated contracting states:
**AT BE CH DE ES FR GB GR IT LI LU NL SE**

(56) References cited:
EP-A- 0 014 225
EP-A- 0 183 451
EP-A- 0 183 545
EP-A- 0 183 547
DE-C- 767 447
US-A- 4 144 191

(73) Proprietor: **NESTE OY**
**Keilaniemi**
**SF-02150 Espoo 15 (FI)**

(72) Inventor: **Alvila, Leila**
**Mastotie 4 B 11**
**SF-80160 Joensuu (FI)**
Inventor: **Pakkanen, Tapani**
**Koulukatu 34 A 12**
**SF-80120 Joensuu (FI)**
Inventor: **Krause, Outi**
**Kaunispaantie 3 F 59**
**SF-00970 Helsinki (FI)**
Inventor: **Joutsimo, Matteus**
**Riskutie 25 A**
**SF-00950 Helsinki (FI)**

(74) Representative: **Lamb, John Baxter et al**
**MARKS & CLERK 57/60 Lincoln's Inn Fields**
**London WC2A 3LS (GB)**

**EP 0 238 331 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

## Description

The present invention concerns a dual step process for producing isobutylene from propylene and synthesis gas.

$C_4$-olefines are starting materials for numerous petrochemical industries. The greatest part of the total quantity of isobutylene and n-butylenes is consumed in producing petrol components in so-called catalytic polymerizing or alkylation. Ever more often, however, both isobutylene and n-butylenes are used as starting materials for various chemical compounds. Traditionally, butylene rubbers and polyisobutylene are made from isobutylene. The demand of the compound has considerably increased in recent years along with that of methylisobutylene ether (MTBE). The starting materials in the production of MTBE, which is an agent improving the octane number of petrol in high demand, are isobutylene and methanol. The demand of isobutylalcohol (TBA) has also increased for the same reason as that of MTBE. TBA is obtained from isobutylene on hydration. Isobutylene is further consumed, for instance, in the manufacturing of tertiary butylphenols and butylcresols of methylmethacrylate.

Commercially, $C_4$-olefines are mainly produced by two different standard procedures : floating catalytic cracking (FCC), the steam cracking. In the first-mentioned process, petrol components are produced by cracking heavier crude oil fractions. In that connection liquid petroleum gas fractions are formed as by-products, among others. Ethylene is the product in the latter. In addition to their manufacturing as cracking products, it is possible to produce butylenes by dehydrogenating the equivalent paraffins. Dehydrogenation processes are expensive, and one of the essential limiting factors is the availability of the starting materials, above all that of isobutylene. It has also been proposed, see DE-C-767447, to produced isobutylene by dehydrating a mixture of alcohols produced by reacting propylene with synthesis gas in the presence of a cobalt catalyst. US-A-4144191 suggests the use of bimetallic Rh/Co carboxyl clusters in the reaction of propylene with synthesis gas but suggests that the use of a mixture of clusters of the individual metals is not useful.

The present invention offers a new advantageous procedure for producing isobutylene in which propylene is used as starting material.

The process of the invention for producing isobutylene is characterized in that in the first step propylene is contacted with synthesis gas at elevated temperature in the presence of a catalyst system containing a mixture of the monometal cluster compounds $M_4(CO)_{12}$ and $M_2'(CO)_{12}$ where M and M' are Co and Rh, respectively ; whereafter, in the second stage, the alcohol mixture thus obtained is dehydrated to produce a product fraction rich in isobutylene.

The new way to produce $C_4$-olefines is thus to use for starting material $C_3$-olefines. The process consists of two steps : in the first step propylene is hydroformylated to become a mixture of n-butanol and isobutanol, and in the second step the alcohols that have been obtained are dehydrated to become olefines.

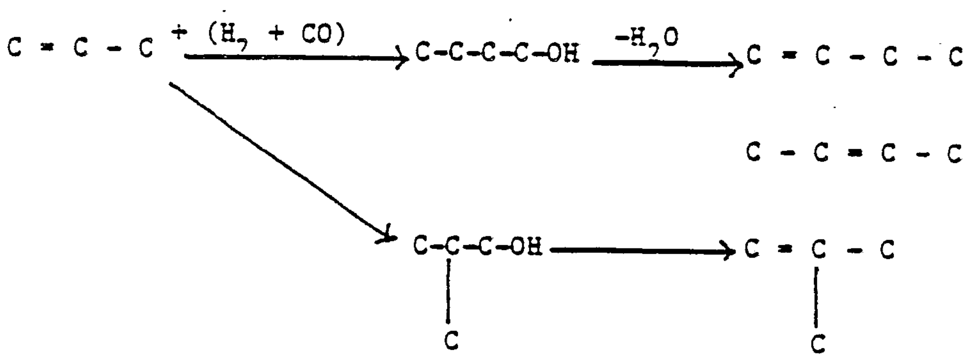


In conventional hydroformylation processes, homogeneous cobalt or rhodium compounds serve as catalyst. One of the most important aims in developing these processes has been highest possible selectivity of compounds with straight chain. It should be noted that the most important products were n-butanol and 2-ethylhexanol, while in the present invention isobutanol is desired. The best, and most active, catalyst for producing n-butanol has turned out to be ligand-modified rhodium carbonyl. With this catalyst, almost exclusively aldehydes are formed (selectivity is typically 96 %) and the proportion of straight-chained and branched is 10-14 :1 (Catalysts in $C_1$ Chemistry, ed. W Keim, 1983). If it is desired to go to olefines from the aldehydes which have been formed, it becomes necessary first to hydrogenate the aldehydes to produce the equivalent alcohols. Modified cobalt catalysts produce alcohols (selectivity 80 %). Their hydrogenating capacity is so good that alkanes are formed with about 15 % selectivity.

In the isobutylene producing process of the invention the newest catalysts are utilized, with which it is possible by hydroformylation to obtain alcohols directly, the selectivity of alcohol formation being 95 %. Such catalysts have been described in the Finnish patent application No. 84 4634 and in the U.S. Patent

No. 4,144,191. The proportion of straight-chain and branched products is also different from those obtained with commercial products, varying in the range of 1.1-1.3 :1, that is, the proportion of iso forms is considerably higher. Since in the developing of commercial hydroformylation processes maximum selectivity in favour of straight-chain fractions was important and the result of hydroformylation mainly consisted of aldehydes, which had to be hydrogenated in order to obtain an alcohol, it has not been possible heretofore to produce selectively in two steps a product fraction with high isobutylene content, applying commercial catalysts.

In the process of the invention, cluster compounds formed by rhodium and cobalt serve as catalysts, in which the ligands typically consist of carbonyl groups. The preparation of the catalysts has been described in the patents cited above. The process conditions are typically : total pressure 20-55 bar, hydrogen/carbon monoxide proportion 1 :1, and temperature 50-200 °C.

In hydroformylation, using the above-mentioned catalyst, alcohol mixtures are obtained which contain about 50 % n-butanol and about 50 % isobutanol.

Decomposing alcohols to olefines by dehydrating is a process known in the art. The process is catalyzed by acids. Typical catalysts are : activated aluminium oxide, phosphoric acid on a carrier, zinc oxide on aluminium oxide carrier, and clay, aluminium silicate, zeolites or ion exchange resins.

The process conditions applied depend on the catalysts and starting materials. Tertiary alcohols for instance are dehydrated with considerably greater ease than primary and secondary alcohols. Conditions also have an effect on selectivity. In addition to olefines also ethers are produced from alcohols especially at the lowest temperatures, particularly from those having a straight chain. It is possible that n-butanol alone is converted to butylenes. In that case a 5A molecular sieve constitutes the catalyst. Isobutanol will then fail to react and can be used for instance as a solvent or as a petrol component.

When carrying out dehydration with aluminium oxide catalyst one has to keep the temperature typically over 300 °C in order that the formation of ethers might be minimized.

The invention is further described with the following examples.

Example 1

The catalyst for use in the hydroformylation reaction was prepared by mixing together 30 mg of the monometal compound $Co_4(CO)_{12}$, 57 mg of the monometal compound $Rh_4(CO)_{12}$ and 125 mg aminic ion exchange resin Dowex MWA-P, and 10 ml toluene, mixed in nitrogen atmosphere for 18 hours. The toluene containing unbound clusters was removed and the catalyst dried in vacuum.

Hydroformylation of propylene took place with 50 bar starting pressure. The partial pressures were : $p_H = 20$ bar, $p_{CO} = 25$ bar, and $p_C = 5$ bar. The temperature was 100 °C, the catalyst quantity 100 mg and the reaction time, 17 hrs.

The reaction product contained butanols 98.9 % and aldehydes 1.1 %. Among the alcohols, the proportion of straight-chained to branched alcohols was 1.3.

Dehydration of the alcohols was carried out in a continuous-action, solid bed reactor. The catalyst was aluminium oxide (Harshaw Al-3996 R). Process conditions were : pressure = 1 bar, temperature = 300 °C, and LHSV = 1.5 hrs.

Under these conditions the alcohol conversion was 100 % and the composition of the product therefrom obtained was : 1-butylene 33 %, 2-butylenes 19 %, isobutylene 48 %, and ethers 0.3 %.

Example 2

In the hydroformylation reaction a catalyst was employed which was prepared by mixing together 1.0 g aluminium oxide (Alumina grades D, dried at 800 °C) and a cluster compound mixture containing 0.035 g $Co_4(CO)_{12}$ (Strem Chemicals) and 0.071 g $Rh_4(CO)_{12}$ (Martinego, S. et al., Inorganic Synthetis, vol. 20, 1980, p. 209), and 0.020 $dm^3$ hexane, in nitrogen atmosphere for 16 hrs. The hexane containing unbound cluster was removed. The catalyst was rinsed with hexane and dried in vacuum.

Propylene hydroformylation was carried out under 54 bar pressure. The partial pressures were : hydrogen 25 bar, carbon monoxide 25 bar, and propylene 4 bar. The catalyst quantity was 350 mg, temperature 100 °C and reaction time 17 hrs.

The liquid product contained alcohols 88 % and aldehydes 7 %. Among the butanols, the straight-chain/branched proportion was 1.1.

Alcohol dehydration was carried out as in Example 1. The product contained 48 % isobutylene.

**Claim**

A two step process for producing isobutylene from propylene and synthesis gas, characterized in that in the first step propylene is contacted with synthesis gas at elevated temperature in the presence of a catalyst comprising a mixture of monometal compounds $M_4(CO)_{12}$ and $M_4'(CO)_{12}$ wherein, M and M' are Co and Rh, respectively, and, in the second step, the alcohol mixture thus obtained is dehydrated to produce a product fraction rich in isobutylene.

**Patentanspruch**

Zweistufiges Verfahren zur Herstellung von Isobutylen aus Propylen und Synthesegas, dadurch gekennzeichnet, dass im ersten Schritt Propylen bei hoher Temperatur mit Synthesegas in Gegenwart eines eine Mischung von monometallischen Verbindungen $M_4(CO)_{12}$ und $M_4'(CO)_{12}$ enthaltenden Katalysators in Kontakt gebracht wird, wobei M and M' jeweils Co and Rh bedeuten, und im zweiten Schritt die so erhaltene Alkoholmischung dehydratiert wird, um eine an Isobutylen reiche Produktefraktion zu ergeben.

**Revendication**

Un procédé en deux étapes pour la production d'isobutylène à partir de propylène et de gaz de synthèse, caractérisé en ce que, dans la première étape, le propylène est mis au contact de gaz de synthèse à température élevée en présence d'un catalyseur, comprenant un mélange des composés monométalliques $M_4(CO)_{12}$ et $M_4'(CO)_{12}$, dans lesquels M et M' sont respectivement Co et Rh, et dans la seconde étape, le mélange d'alcools ainsi obtenu est déshydraté pour former une fraction de produit riche en isobutylène.